# EUROPEAN PATENT APPLICATION

(11) **EP 4 725 432 A1**
(43) Date of publication of application: **15.04.2026**
(21) Application number: 24819161.1
(22) Date of filing: 22.05.2024
(51) Int. Cl.: A61B 18/12

(54) **HIGH-FREQUENCY TREATMENT DEVICE AND HIGH-FREQUENCY TREATMENT METHOD**

(30) Priority: 07.06.2023 JP 2023093662
(71) Applicant: Kabushiki Kaisha Top, Adachi-ku, Tokyo 120-0035 (JP)
(72) Inventor: IKEUCHI, Atsushi, Tokyo 120-0035 (JP)
(74) Representative: Sekiguchi, Kazuya
(86) International application number: PCT/JP2024/018824
(87) International publication number: WO 2024/252928

(57) **Abstract**

[Problem] To provide a high-frequency treatment apparatus and a high-frequency treatment method capable of efficiently heating a wide region.

[Solution] A high-frequency treatment apparatus comprises : a plurality of high-frequency output units 40 that output high-frequency power; a plurality of electrodes 20 that are connected to the high-frequency output units 40 and are positioned on an object to be treated; a switching unit 90 that is provided between the high-frequency output units 40 and the electrodes 20, and switches the flow of a high-frequency current to either an electrode set 200 or an electrode set 210, the electrode sets 200, 210 each including a combination of at least two electrodes 20; and a control unit that controls the switching unit 90 so as to periodically switch between a first connection state Sq1 in which different electrode sets 200 are connected to the respective high-frequency output units 40 during the treatment and a second connection state Sq2 in which an electrode set 210 obtained by combining the plurality of electrode sets 200 in the first connection state Sq1 is connected to one high-frequency output unit 40.

## Description

### [Technical field]

The present invention relates to a high-frequency treatment apparatus and a high-frequency treatment method for performing various treatments by passing a high-frequency current to a treatment object.

### [Background art]

Conventionally, radiofrequency treatment has been widely used in the field of medicine, in which electrodes are placed in the bodies of humans and animals, and radiofrequency current is applied from these electrodes to ablate tissues. In recent years, nerve block using radiofrequency treatment has attracted attention as a pain treatment. Compared with conventional methods using drugs, nerve block using radiofrequency treatment has the advantages of having fewer side effects on surrounding tissues and lasting effects.

There are two types of nerve block using radiofrequency treatment: radiofrequency thermocoagulation and pulsed radiofrequency. In the radiofrequency thermocoagulation method, a part of nerve tissue is heated at a temperature of about 80° C for several minutes by radiofrequency current flowing from an electrode to thermocoagulate and thereby block pain signals. In the pulsed radiofrequency method, a part of nerve tissue is heated at a temperature of 42° C or less for 10 minutes or more by radiofrequency current flowing completely to block pain signals without damaging the nerve.

In such a nerve block, not only a part of 1 nerve is heated to a specific point, but several nerves are heated together. For example, when nerve block for sacroiliac joint pain is performed by high-frequency treatment, it is necessary to heat a large area of the lateral branch of the posterior branch of the sacral nerve (For example, see Patent Document 1).

Therefore, in the conventional method, as shown in FIGS. 1 and 2 of Patent Document 1, a plurality of needles (needles) are punctured near the lateral branch, electrodes are inserted into the needles, and as shown in FIG. 5 of Patent Document 1, high-frequency current is passed between these electrodes and a counter electrode plate arranged on the skin surface of the patient (so-called monopolar) or high-frequency current is passed between two adjacent electrodes (so-called bipolar).

### [Prior art literature]

### [Patent literature]

Patent document 1: Japanese Patent No. 2018-511444

### [Outline of the invention]

### [Problem to be solved by the invention]

However, in the conventional monopolar device, only one electrode is used for one treatment, and the area that can be heated by one electrode is also narrow. Therefore, in order to heat a wide area, it is necessary to perform a plurality of treatments by replacing electrodes, which requires time and labor. In addition, in the bipolar device, the area between two electrodes and the surrounding area is heated, so that a wider area can be heated by one treatment than in the monopolar device. However, in order to perform nerve block for sacroiliac joint pain, it is necessary to perform a plurality of treatments by replacing electrodes.

It is an object of the present invention to provide a high-frequency treatment apparatus and a high-frequency treatment method that can efficiently heat a wide area.

### [Solution to Solve the Problem]

The high-frequency treatment apparatus comprises: a plurality of high-frequency output units for outputting high-frequency power; a plurality of electrodes connected to the high-frequency output units and arranged on a treatment object; a switching unit for switching which of the electrode sets, which are provided between the high-frequency output units and the electrodes and are composed of at least two of the electrodes, a high-frequency current flows; and a control unit for controlling the switching unit so as to switch between a first connection state in which the electrode sets different for each of the high-frequency output units are connected and a second connection state in which the electrode sets composed of a plurality of the electrode sets in the first connection state are connected to one of the high-frequency output units at a specific cycle.

The high-frequency treatment method according to the present invention disposes a plurality of electrodes on an object to be treated, and performs treatment by passing high-frequency currents from a plurality of high-frequency output units to any one of the electrode sets obtained by combining at least two of the electrodes, wherein a first connection state in which the electrode sets different for each of the high-frequency output units are connected and a second connection state in which the electrode sets obtained by combining the plurality of electrode sets in the first connection state are connected to one of the high-frequency output units are connected at a specific cycle during treatment.

According to the high-frequency treatment apparatus and the high-frequency treatment method according to the present invention, the heating state in the first connection state and the heating state in the second connection state can be made to compensate for portions with low heating degrees, so that a wide area can be efficiently heated.

In the high-frequency treatment apparatus of the present invention, the second connection state is preferably a connection state in which the electrodes included in the same electrode set in the first connection state have the same polarity.

According to this, the degree of double heating in the first connection state and the second connection state can be reduced, and uneven heating can be reduced, so that a wide area can be efficiently heated.

In the high-frequency treatment apparatus of the present invention, the second connection state is preferably a connection state in which the electrode set including all the electrodes is connected to one of the high-frequency output sections.

According to this, it is possible to comprehensively heat between and around a plurality of electrodes, so that a wide area can be efficiently heated.

Preferably, the high-frequency treatment apparatus according to the present invention includes an impedance measurement unit which is connected to the electrodes via the switching unit and measures impedance by outputting an impedance measurement signal, and the control unit controls the switching unit so as to provide a third connection state for connecting the electrode set to the impedance measurement unit between the first connection state and the second connection state.

According to this configuration, while the switching between the first connection state and the second connection state needs to be performed during a stop period for stopping the output of high-frequency power, high-precision impedance measurement can be performed by the dedicated impedance measurement unit by utilizing this stop period, so that a wide area can be efficiently heated.

### [Effect of the invention]

According to the high-frequency treatment apparatus and the high-frequency treatment method according to the present invention, an excellent effect of efficiently heating a wide area can be obtained.

### [Brief Description of the Drawings]

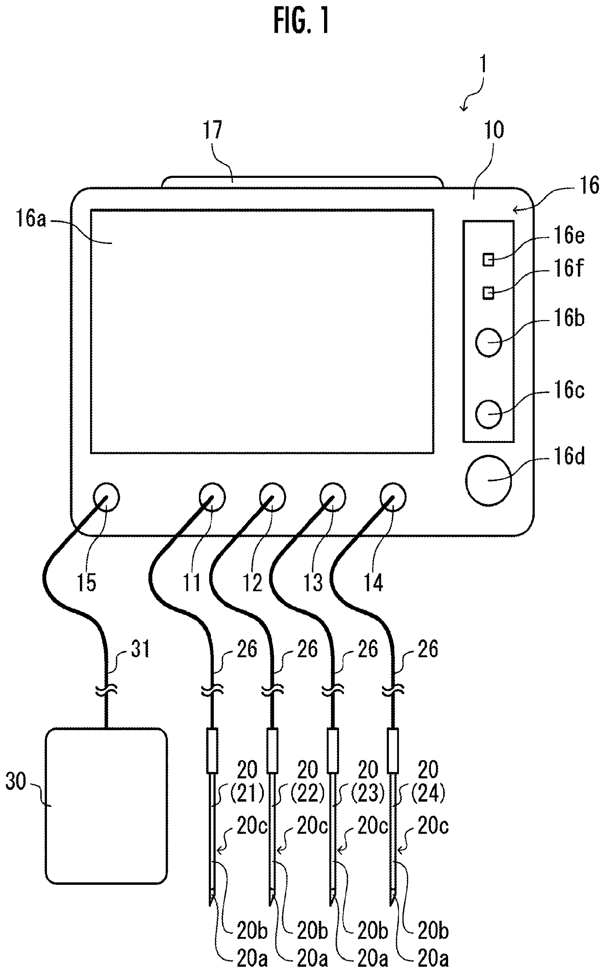

[Fig. 1] FIG. 1 is a schematic diagram showing an external appearance of a high-frequency treatment apparatus according to an embodiment of the present invention;

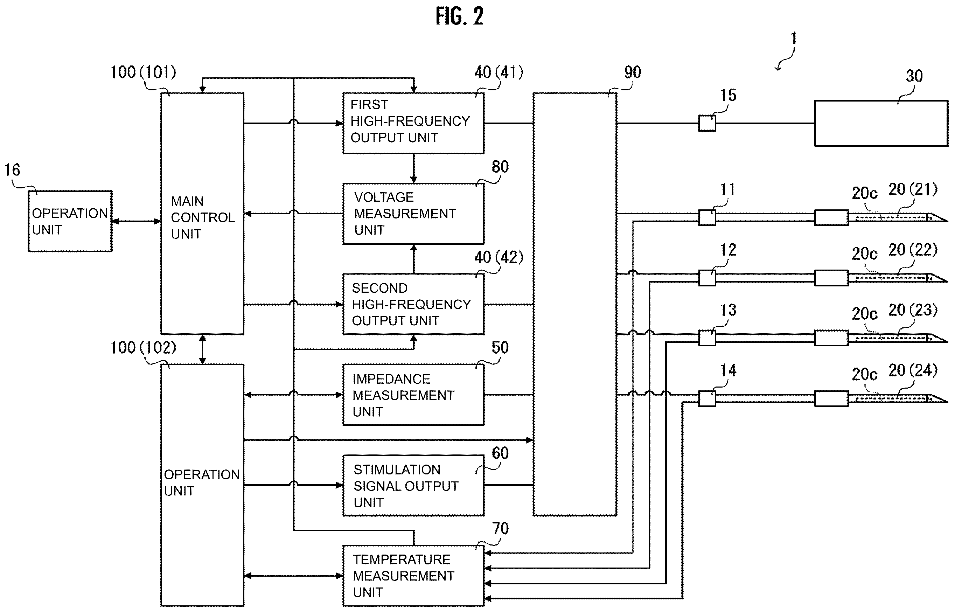

[Fig. 2] FIG. 2 is a block diagram schematically showing an internal configuration of a high-frequency treatment apparatus;

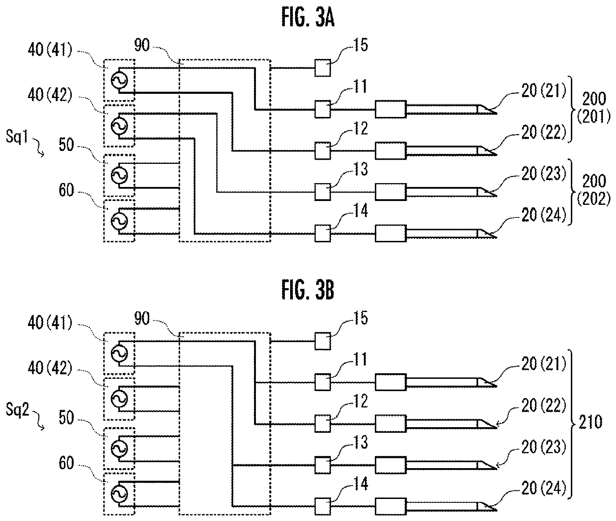

[Fig. 3] FIGS. 3A and 3B are schematic diagrams showing a connection state when a high-frequency thermal solidification method is performed with a quad-polar output type;

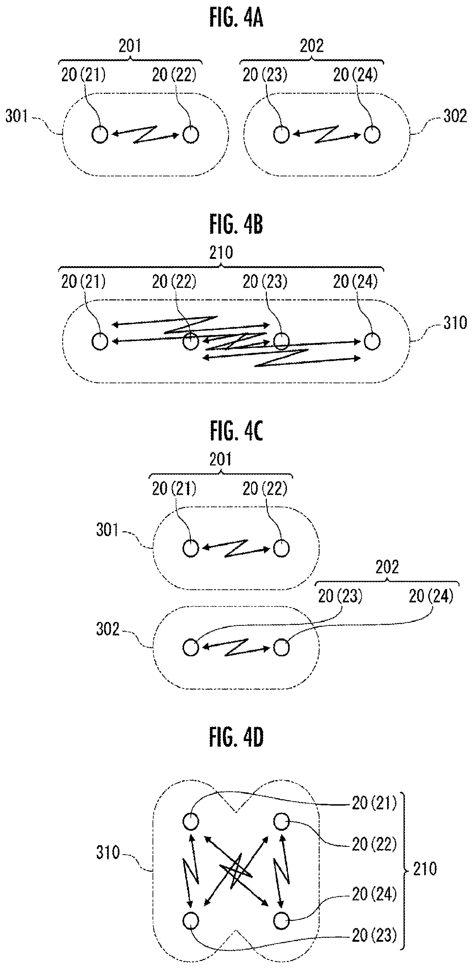

[Fig. 4] FIGS. 4A to 4D are schematic diagrams schematically showing how a high-frequency current flows in a treatment object;

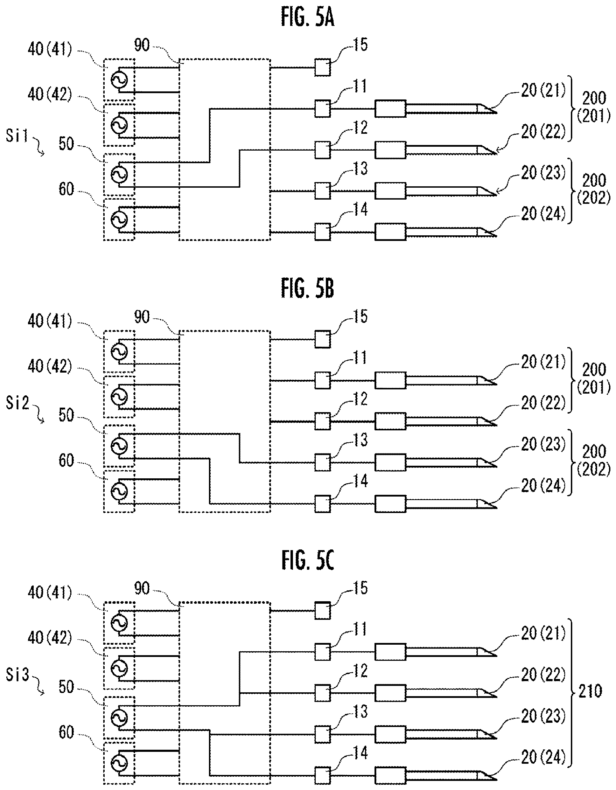

[Fig. 5] FIGS. 5A to 5C are schematic diagrams showing a connection state for measuring impedance when the output type is a quad-polar output type;

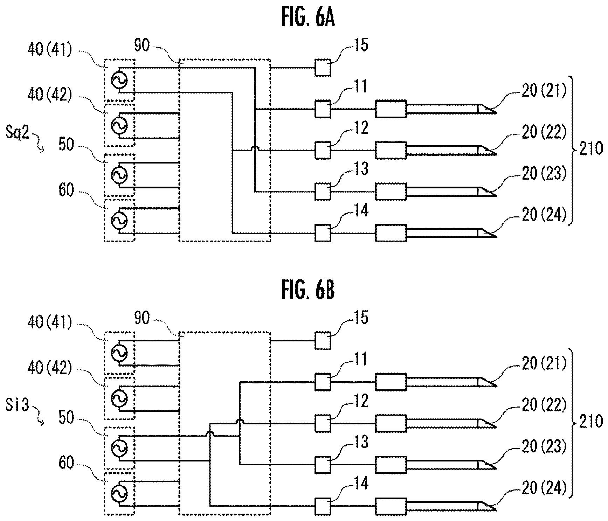

[Fig. 6] FIGS. 6A and 6B are schematic diagrams showing an example when electrodes included in the same first electrode set in the first connection state do not have the same polarity in the second connection state;

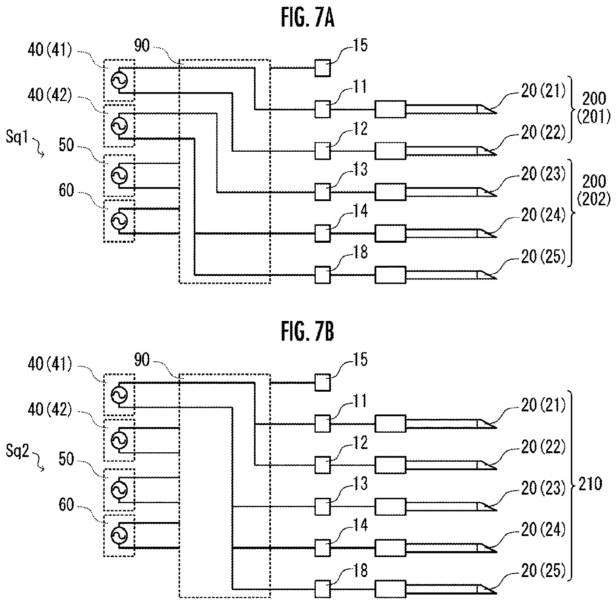

[Fig. 7] FIGS. 7A and 7B are schematic diagrams showing an example when five or more electrodes can be used;

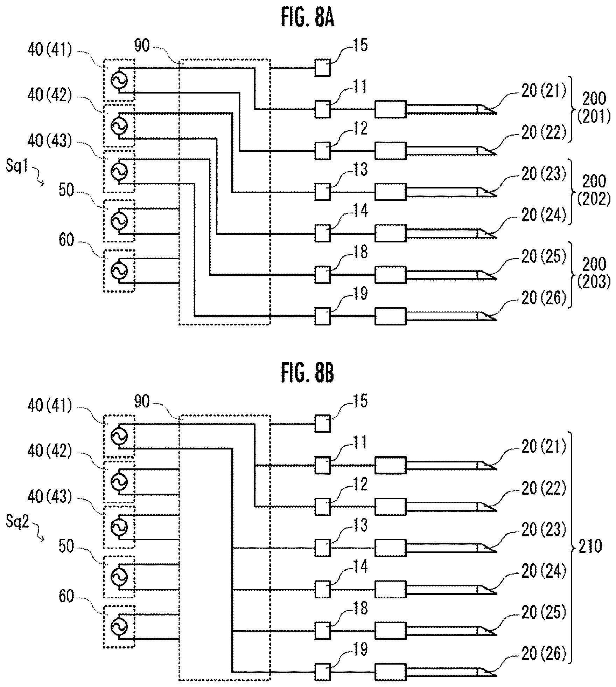

[Fig. 8] FIGS. 8A and 8B are schematic views showing an example in which three or more high-frequency output units are provided.

### [Embodiment of the invention]

Hereinafter, an embodiment of the present invention will be described with reference to the accompanying drawings.

FIG. 1 is a schematic view showing an appearance of a high-frequency treatment apparatus 1 according to an embodiment of the present invention. The high-frequency treatment apparatus 1 according to the present embodiment applies a high-frequency voltage to a living body such as a human being or an animal to be treated, flows a high-frequency current in the living body, and performs treatment by either a high-frequency thermocoagulation method or a pulsed high-frequency method. As shown in FIG. 1, the high-frequency treatment apparatus 1 includes a body 10, 4 electrodes 20(21, 22, 23, and 24) connected to the body 10, and a counter electrode plate 30 connected to the body 10.

The main body 10 accommodates or supports internal structures to be described later. On the front surface of the main body 10, 4 electrode connectors 11-14 to which electrodes 20 are connected and a counter electrode plate connector 15 to which a counter electrode plate 30 is connected are provided at a lower portion. On the front surface of the main body 10, an operation section 16 for receiving an operation by a user is also provided. The operation section 16comprises a touch panel display 16a for receiving input operations such as various settings and displaying various types of information, a start button 16b for receiving a start operation of a treatment, a stop button 16c for receiving an end operation of a treatment, a control knob 16d for performing an output start operation and an output voltage adjustment operation during manual operation, an output indicator 16e which is lit during the output of high-frequency power, and an alarm indicator 16f which is lit when an alarm is generated.

On the upper portion of the main body 10, a handle 17 for carrying the main body 10 is provided. Although not shown, a power connector connected to a commercial AC power source for receiving power, a power switch for receiving a power-on operation, and a USB connector for connecting an external storage means are provided on the rear surface of the main body 10. A speaker (alarm) for outputting a buzzer sound is provided in the body 10 together with a drive circuit.

The electrode 20 is inserted into an object to be treated in a high-frequency treatment, and a high-frequency current flows in the object to be treated. In this embodiment, the electrode 20 is formed into a needle tube that can be directly punctured into a human body or the like. Most of the electrode 20 is an insulating portion 20b coated with insulation except for the non-insulating portion 20a at the tip, and the high-frequency current is output from the non-insulating portion 20a. The electrode 20 also incorporates a thermocouple 20c for measuring the temperature around the electrode 20 during the treatment.

The electrode 20 is electrically connected to the main body 10 via an electrode cable 26 and an electrode connector 11-14. In this embodiment, a maximum of 4 electrodes 20 (21-24) can be used simultaneously. The electrodes 20 may have other shapes, such as rod-shaped electrodes inserted into a needle tube or catheter, or pad-shaped electrodes arranged on the surface of a living body. The thermocouple 20c may be provided separately from the electrodes 20.

The counter electrode plate 30 is a plate-shaped electrode which is attached to the surface of the object to be treated and is used for passing a high-frequency current to and from the electrode 20. In this embodiment, it is possible to perform treatment by passing a high-frequency current between the electrodes 20 and the counter electrode plate 30 or between the electrodes 20 (for example, between the electrodes 21 and 22). The counter electrode plate 30 is electrically connected to the main body 10 via the counter electrode plate cable 31 and the counter electrode plate connector 15. In this embodiment, the counter electrode plate 30 is formed in a rectangular flat plate shape, but the counter electrode plate 30 may have other shapes.

FIG. 2 is a block diagram schematically showing the internal configuration of the high-frequency treatment apparatus 1. As shown in FIG. 2, the high-frequency treatment apparatus 1 includes a high-frequency output unit 40 (a first high-frequency output unit 41 and a second high-frequency output unit 42), an impedance measurement unit 50, a stimulation signal output unit 60, a temperature measurement unit 70, a voltage measurement unit 80, and a control unit 100 (a main control unit 101 and a sub control unit 102).

The high-frequency output unit 40 generates and outputs high-frequency power of a preset frequency (For example, 470-490 kHz) and a voltage (For example, 18-80Vrms) based on a control signal from the main control unit 101 based on power supplied from a commercial AC power source. In this embodiment, by providing two high-frequency output units 40, that is, a first high-frequency output unit 41 and a second high-frequency output unit 42, it is possible to flow a high-frequency current while stably performing output control at the two electrode sets (For example, an electrode set of electrodes 21 and 22 and an electrode set of electrodes 23 and 24) at the same time.

The high-frequency output unit 40 is connected to an electrode connector 11-14 and a counter electrode plate connector 15 via a switching unit 90. Accordingly, the electrode 20 is connected to the high-frequency output section 40 via the electrode connector 11-14 and the switching section 90, and the counter electrode 30 is connected to the high-frequency output section 40 via the counter electrode connector 15 and the switching section 90. The high-frequency output section 40 is composed of known circuits each having a transformer, whereby the treatment object is insulated from a commercial AC power supply.

The impedance measurement section 50 is composed of known circuits and the like, generates and outputs an impedance measurement signal composed of relatively weak AC power, and measures the impedance between the electrode 20 and the counter electrode 30 or between the electrodes 20, that is, the impedance of a portion where a high-frequency current flows in the treatment object. The impedance measurement section 50 is connected to the electrode connector 11-14 and the counter electrode connector 15 via the switching section 90. Accordingly, the electrode 20 is connected to the impedance measurement section 50 via the electrode connector 11-14 and the switching section 90, and the counter electrode 30 is connected to the impedance measurement section 50 via the counter electrode connector 15 and the switching section 90.

The impedance measurement section 50 outputs an impedance measurement signal under the control of the sub-control section 102, samples the impedance measurement signal flowing in the treatment object at a predetermined sampling period, discretizes the signal, and calculates the values of the real part and the imaginary part of the complex impedance by performing a discrete Fourier transform. These calculated values are transmitted to the sub-control unit 102. In this embodiment, the impedance measurement signal is a sine wave with a frequency of 50 kHz, the maximum output current is set to about 4 mA, and the maximum output voltage is set to about 500 mVrms. Although the details will be described later, in this embodiment, the intermittent output of the impedance measurement signal is continued before and after the start of the treatment, so that the high-frequency treatment can be efficiently performed.

The stimulation signal output unit 60 is composed of a known circuit or the like, and generates and outputs a stimulation signal for nerve exploration. Usually, before the high-frequency treatment is performed, nerve exploration is performed, and the presence or absence of nerves (sensory nerves and motor nerves) around the electrode 20 is explored. The stimulation signal output unit 60 is connected to the electrode connector 11-14 and the counter electrode plate connector 15 via the switching unit 90. Therefore, the electrode 20 is connected to the stimulation signal output unit 60 via the electrode connector 11-14 and the switching unit 90, and the counter electrode plate 30 is connected to the stimulation signal output unit 60 via the counter electrode plate connector 15 and the switching unit 90.

The stimulation signal output unit 60 operates under the control of the sub-control unit 102, and sends a stimulation signal into the treatment object via the electrode 20. The user confirms the presence of a sensory nerve around the electrode 20 when the treatment object perceives the stimulation signal, and confirms the presence of a motor nerve around the electrode 20 when the muscle of the treatment object contracts in response to the stimulation signal. In this embodiment, the stimulation signal is a bipolar square wave having a predetermined pulse width, and is intermittently output at a predetermined frequency.

The temperature measuring unit 70 is composed of a known circuit or the like, and measures the temperature around the electrode 20 by the thermocouple 20c. In this embodiment, the temperature measuring unit 70 is connected to the thermocouple 20c in the electrode 20 via the electrode connector 11-14, but a connector for the thermocouple 20c may be provided separately.

The temperature measurement unit 70 operates under the control of the sub-control unit 102, generates a temperature measurement signal (E.G. 25 mV/° C) based on the signal received from the thermocouple 20c, and transmits it to the sub-control unit 102. The temperature measurement unit 70 also outputs an output stop signal to the high-frequency output unit 40 and the main control unit 101 when a temperature abnormality occurs (when the temperature measurement value is 7° C more or higher than the set temperature). The high-frequency output unit 40 which has received the output stop signal stops outputting the high-frequency power. The main control unit 101 which has received the output stop signal turns on the alarm indicator 16f and outputs an alarm buzzer sound from a speaker.

The voltage measurement unit 80 is composed of a known circuit or the like and measures the voltage of the high-frequency power output from the high-frequency output unit 40. The voltage measurement unit 80 individually measures the voltage of the high-frequency power output from the first high-frequency output unit 41 and the second high-frequency output unit 42, generates a voltage measurement signal based on the voltage measurement signal, and transmits the voltage measurement signal to the main control unit 101.

The switching unit 90 comprises a plurality of lead relays, semiconductor switches, or the like, and switches the connection states of the first high-frequency output unit 41, the second high-frequency output unit 42, the impedance measurement unit 50, and the stimulation signal output unit 60 with the electrode connector 11-14 and the counter electrode plate connector 15.

Specifically, the switching unit 90 operates under the control of the sub-control unit 102 to switch which of the first high-frequency output unit 41, the second high-frequency output unit 42, the impedance measurement unit 50, and the stimulation signal output unit 60 is connected to the electrode connector 11-14 and the counter electrode plate connector 15. That is, the switching unit 90 switches which of the high-frequency power, the impedance measurement signal, and the stimulation signal is output to the electrode 21-24 and the counter electrode plate 30 connected to the electrode connector 11-14 and the counter electrode plate connector 15. The switching unit 90 also operates under the control of the sub-control unit 102 to switch which combination of the electrode 21-24 and the counter electrode plate 30 connected to the electrode connector 11-14 and the counter electrode plate connector 15 (That is, electrode sets) the high-frequency current, the impedance measurement signal, and the stimulation signal flows.

The control unit 100 executes the high-frequency treatment by controlling each part of the high-frequency treatment apparatus 1. In this embodiment, the control unit 100 comprises a main control unit 101 for controlling the high-frequency output unit 40 and the operation unit 16, and a sub-control unit 102 for controlling the impedance measurement unit 50, the stimulation signal output unit 60, the temperature measurement unit 70, and the switching unit 90. In this manner, the sub-control unit 102, controlling a portion provided closer to the treatment target than the high-frequency output unit 40, is provided separately from the main control unit 101 and it is possible to more reliably insulate the treatment target from the commercial AC power supply.

The main control unit 101 has a known configuration such as a CPU, a ROM, a RAM, and an auxiliary storage device. The main control unit 101 starts and ends the high-frequency treatment based on an input operation received by the operation unit 16 and information stored in the ROM or the like. The main control unit 101 controls the output of the high-frequency output unit 40 during the treatment. Specifically, the main control unit 101 controls the output so that the temperature measurement value received from the sub-control unit 102 becomes substantially equal to the set temperature by using the output voltage as the operation quantity in the high-frequency thermal solidification method and the output pulse width as the operation quantity in the pulsed high-frequency method (The output voltage is fixed to the voltage set value.). In both the high-frequency thermal solidification method and the pulsed high-frequency method, the main control unit 101 controls the ON/OFF of the output of the high-frequency power at a set cycle.

During the processing, the main control unit 101 causes the touch panel display 16a to display information such as elapsed time, temperature measurement value, impedance measurement value, voltage measurement value, and current measurement value. In this embodiment, the current measurement value is calculated from the voltage measurement value and the impedance measurement value by the main control unit 101.

The sub-control unit 102 has a known configuration such as a CPU, ROM, and RAM. The sub-control unit 102 calculates a temperature measurement value based on the temperature measurement signal received from the temperature measurement unit 70, and transmits it to the main control unit 101. The sub-control unit 102 also calculates an impedance measurement value based on the values of the real part and imaginary part of the complex impedance received from the impedance measurement unit 50 and a calibration value generated in advance, and transmits it to the main control unit 101.

This calibration value is generated by the sub-control unit 102 every time the high-frequency treatment apparatus 1 is started. When the power of the high-frequency treatment apparatus 1 is turned on, the sub-control unit 102 causes the impedance measuring unit 50 to measure the impedance of the built-in fixed resistance of 100 Ω and the fixed resistance of 1 kΩ. Then, a low-resistance calibration value is generated based on the measurement result of the fixed resistance of 100 Ω, and a high-resistance calibration value is generated based on the measurement result of the fixed resistance of 1 kΩ, and stored in the RAM. The impedance measurement value is calculated by using the high-resistance calibration value in the first calculation, and in subsequent calculations, a predetermined value (300 Ω or 400 Ω) is set as a threshold value, and either the low-resistance calibration value or the high-resistance calibration value is used based on the previous calculation result.

Further, the sub-control unit 102 controls ON/OFF of a plurality of switches provided in the switching unit 90, and switches the connection states of the first high-frequency output unit 41, the second high-frequency output unit 42, the impedance measuring unit 50, and the stimulation signal output unit 60 with the electrode connector 11-14 and the counter electrode plate connector 15. Specifically, the sub-control unit 102 makes the switching unit 90 switch the connection states based on the connection states of the electrode 20 and the counter electrode plate 30 detected from the electrode connector 11-14 and the counter electrode plate connector 15, and mode information and output format information received from the main control unit 101.

In this embodiment, there are four operation modes: a menu mode in which various settings are performed, a stimulation mode in which a stimulation signal is output to perform nerve exploration, a region mode in which a high-frequency thermal solidification method is performed by outputting high-frequency power, and a PRF mode in which a pulsed high-frequency method is performed by outputting high-frequency power. There are 4 types of output types: monopolar with at least one of the electrodes 21-24 and the counter electrode plate 30 as an electrode set, bipolar with the electrodes 21 and 22 or the electrodes 23 and 24 as an electrode set, tripolar with the electrode 21-23 as an electrode set, and quad-polar with the electrode 21-24 as an electrode set.

Next, an operation in the case of performing the high-frequency thermal solidification method using quad-polar as an output type, which is a feature of this embodiment, will be described.

FIGS. 3A and 3B are schematic diagrams showing a connection state in the case of performing the high-frequency thermal solidification method using quad-polar as an output type. Before starting the high-frequency thermal solidification method, various checks, arrangement of the electrode 20 to the treatment object, and nerve exploration by the arranged electrode 20 are performed. The main control unit 101 starts the treatment based on the fact that the set time and set temperature are set in the region mode and the start operation is received by the start button 16b or the control knob 16d.

During the process, when the output format is quad-polar, the sub-control unit 102 controls the switching unit 90 so as to switch the connection state between the high-frequency output unit 40 and the electrodes 20 at a specific cycle (In this embodiment, 100msec). Specifically, the sub-control unit 102 controls the switching unit 90 so as to switch the connection state between the first connection state Sq1 shown in FIG. 3A and the second connection state Sq2 shown in FIG. 3B.

The first connection state Sq1 is a connection state in which different first electrode sets 200 are connected for each of the two high-frequency output units 40. In the present embodiment, in the first connection state Sq1, the first electrode set 201 consisting of the electrodes 21 and 22 is connected to the first high-frequency output unit 41, and the first electrode set 202 consisting of the electrodes 23 and 24 is connected to the second high-frequency output unit 42.

The second connection state Sq2 is a connection state in which the second electrode set 210 formed by combining a plurality of first electrode sets 200 is connected to one high-frequency output unit 40. In this embodiment, in the second connection state Sq2, the second electrode set 210(That is, the combined electrode set of the first electrode sets 201 and 202) comprising the electrodes 21-24 is connected to the first high-frequency output section 41. In the second connection state Sq2, the electrodes 21 and 22 have the same polarity (connected to the same output terminal) by being shorted to each other, and the electrodes 23 and 24 have the same polarity.

In this embodiment, by switching the first connection state Sq1 and the second connection state Sq2 at a specific cycle as described above, it is possible to efficiently heat a wide area in the high-frequency heat solidification method. FIGS. 4A to 4D are schematic diagrams schematically showing how a high-frequency current flows in a treatment object, and the electrodes 20 arranged in the treatment object are viewed in the axial direction.

FIGS. 4A and 4B show a case where the electrodes 21-24 are arranged in a row. In this case, in the first connection state Sq1, the high-frequency current flows between the electrodes 21 and 22, and the high-frequency current flows between the electrodes 23 and 24, as shown in FIG. 4A. As a result, the treatment target is heated in the region 301 between and around the electrodes 21 and 22, and in the region 302 between and around the electrodes 23 and 24. Further, depending on the distance between the electrodes 22 and 23, as shown in FIG. 4A, there is a region between the region 301 and the region 302 in which no heating occurs.

In the second connection state Sq2, as shown in FIG. 4B, a high-frequency current flows between the electrodes 21 and 23, between the electrodes 21 and 24, between the electrodes 22 and 23, and between the electrodes 22 and 24. As a result, the treatment target is heated in the region 310 between and around the electrodes 21-24. However, in this region 310, since more high-frequency current flows between the electrodes 22 and 23, the region between and around the electrodes 22 and 23 is heated more intensively than other regions. In addition, the degree of heating between and around the electrodes 21 and 22, and between and around the electrodes 23 and 24 is reduced compared with that in the first connection state Sq1.

Therefore, by passing the high-frequency current in the first connection state Sq1 and then passing the high-frequency current in the second connection state Sq2, the heating state in the first connection state Sq1 is complemented, and the area between and around the electrodes 21-24 can be heated substantially uniformly.

FIGS. 4C and 4D show a case where the electrodes 21-24 are arranged in two rows. In this case, as shown in FIG. 4C, in the first connection state Sq1, the treatment object is heated in the area 301 between and around the electrodes 21 and 22, and in the area 302 between and around the electrodes 23 and 24. Further, depending on the distance between the electrodes 21 and 23, and the distance between the electrodes 22 and 24, a region between the area 301 and the area 302 is not heated.

In the second connection state Sq2, as shown in FIG. 4D, a high-frequency current flows comprehensively between the electrodes 21 and 22 and the electrodes 23 and 24. Therefore, also in this case, by flowing the high-frequency current in the first connection state Sq1 and then flowing the high-frequency current in the second connection state Sq2, the heating state in the first connection state Sq1 is complemented, and the area between and around the electrodes 21-24 can be heated substantially uniformly.

As described above, in the present embodiment, by alternately repeating the heating in the first connection state Sq1 and the heating in the second connection state Sq2, the heating states in the two connection states are mutually complemented, and it is possible to efficiently heat a wide area between and around the 4 electrodes 21-24 in one treatment.

In particular, in the present embodiment, in the second connection state Sq2, the electrodes 21 and 22 included in the first electrode set 201 are made to have the same polarity, and the electrodes 23 and 24 included in the first electrode set 202 are made to have the same polarity, so that the degree of double heating of the regions 301 and 302 is reduced, and uneven heating is more difficult to occur. Furthermore, by reducing the degree of double heating in this way, the frequency of intervention of output control (reduction of output voltage) for lowering the temperature is also reduced, so that more efficient heating is possible.

In the present embodiment, in the second connection state Sq2, all the electrodes 21-24 are included in one second electrode set 210, so that it is possible to comprehensively heat a wide area between and around all the electrodes 21-24.

During the switching operation of the switching unit 90 for switching between the first connection state Sq1 and the second connection state Sq2, the output of the high-frequency power from the first high-frequency output unit 41 and the second high-frequency output unit 42 is stopped. Specifically, in this embodiment, a stop period of 9msec for stopping the output of the high-frequency power from the first high-frequency output unit 41 and the second high-frequency output unit 42 is provided at a cycle of 100msec, and the switching operation of the switching unit 90 is performed during this stop period.

In this embodiment, the impedance measurement unit 50 measures the impedance during this stop period, so that the processing can be performed more efficiently. FIGS. 5A to 5C are schematic diagrams showing connection states for measuring the impedance when the output format is quad-polar.

Specifically, during a stop period between the first connection state Sq1 and the second connection state Sq2, the sub-control unit 102 controls the switching unit 90 so that the third connection state Si1, Si2 or Si3 connecting the impedance measurement unit 50 and the electrodes 20 is continued for 5msec. Then, the sub-control unit 102 controls the impedance measurement unit 50 so as to output an impedance measurement signal and measure impedance during this 5msec.

Since the impedance measurement needs to be performed for each of the first electrode sets 201 and 202 and the second electrode set 210, the third connection states Si1 to Si3 are also of three types. That is, in this embodiment, there are three types: the third connection state Si1 (FIG. 5A) for measuring the impedance in the first electrode set 201 by connecting the electrodes 21 and 22 to the impedance measurement unit 50, the third connection state Si2 (FIG. 5B) for measuring the impedance in the first electrode set 202 by connecting the electrodes 23 and 24 to the impedance measurement unit 50, and the third connection state Si3 (FIG. 5C) for measuring the impedance in the second electrode set 210 by connecting the electrodes 21-24 to the impedance measurement unit 50.

During the stop period, when the connection state before the stop period is the first connection state Sq1, the switching unit 90 first switches from the first connection state Sq1 to the third connection state Si1 or Si2, and then 5msec later switches from the third connection state Si1 or Si2 to the second connection state Sq2. Which of the third connection states Si1 and Si2 is switched first is changed for each switch period. When the connection state before the stop period is the first connection state Sq1, the switching unit 90 first switches from the second connection state Sq2 to the third connection state Si3 during the stop period, and then switches from the third connection state Si3 to the first connection state Sq1 after 5msec.

In this embodiment, by measuring the impedance by the impedance measuring unit 50 while utilizing the stop period required for switching between the first connection state Sq1 and the second connection state Sq2 as described above, it is possible to measure the impedance with higher accuracy as compared with the conventional method of calculating the impedance from the voltage measurement value and the current measurement value of the high-frequency power. From the change in the impedance measurement value during the treatment, it is possible to detect a defect such as a positional shift or a falling off of the electrode 20, and by improving the accuracy of the impedance measurement, it is possible to facilitate early detection of such a defect and to efficiently perform heating by the high-frequency current.

Next, a modified example of the high-frequency treatment apparatus 1 will be described.

FIGS. 6A and 6B are schematic views showing an example in which the electrodes 20 included in the same first electrode set 200 in the first connection state Sq1 are not set to have the same polarity in the second connection state Sq2. Specifically, in this example, the electrodes 21 and 23 are set to have the same polarity, and the electrodes 22 and 23 are set to have the same polarity in the second connection state Sq2. The third connection state Si3 in this example is a connection state in which the impedance measuring section 50 replaces the first high-frequency output section 41 in the second connection state Sq2.

As described above, the polarity of the electrodes 21-24 in the second connection state Sq2 is not particularly limited, and an appropriate polarity can be adopted in accordance with the type and state of the part to be heated, the arrangement of the electrodes 21-24, and the like. The polarity of the electrodes 21-24 in the second connection state Sq2 may be changeable before the treatment, or may be changed during the treatment.

FIGS. 7A and 7B are schematic diagrams showing an example in which 5 or more electrodes 20 can be used. Specifically, in this example, the electrode connector 18 and the electrode 25 are added, the first electrode set 201 is composed of the electrode 23-25, and the second electrode set 210 is composed of the electrode 21-25.

As described above, the number of electrodes 20 usable by the high-frequency treatment apparatus 1 and the number of electrodes 20 included in the first electrode set 200 in the first connection state Sq1 are not particularly limited, and any number can be employed. Further, it is needless to say that it is not necessary to use all the electrodes 20 in the treatment, and only a part of the usable electrodes 20 may be used.

FIGS. 8A and 8B are schematic views showing an example in which three or more high-frequency output units 40 are provided. Specifically, in this example, the third high-frequency output unit 43, the electrode connectors 18 and 19, and the electrodes 25 and 26 are added. Further, a first electrode set 203 composed of the electrodes 25 and 26 connected to the third high-frequency output unit 43 is added to the first connection state Sq1, and a second electrode set 210 is composed of the electrodes 21-26.

As described above, the number of the high-frequency output units 40 provided in the high-frequency treatment apparatus 1 is not particularly limited, and any number can be employed together with the electrodes 20, and the number of the first electrode sets 200 can be increased in accordance with the number of the high-frequency output units 40. Further, the second electrode sets 210 need not be a combination of all the first electrode sets 200, and for example, in the examples shown in FIGS. 8A and 8B, the second electrode sets 210 may be composed of the electrodes 21-24. Further, it is needless to say that it is not necessary to use all the high-frequency output units 40 in the treatment.

Although the embodiments of the present invention have been described above, the high-frequency treatment apparatus and the high-frequency treatment method of the present invention are not limited to the above-described embodiments, and various modifications can be made within the scope of the present invention. For example, the high-frequency treatment apparatus and the high-frequency treatment method of the present invention are not limited to performing the high-frequency thermocoagulation method and the pulsed high-frequency method, and may be used for other applications such as, for example, tumor ablation. Further, the object to be treated is not limited to a human body or an animal, and may be other objects.

Further, the operations and effects shown in the above-described embodiments are only a list of the most suitable operations and effects resulting from the present invention, and the operations and effects according to the present invention are not limited thereto.

### [Explanation of reference numerals]

1 High-frequency treatment apparatus
40 High-frequency output section
50 Impedance measurement section
20 Electrode
90 Switching section
100 Control section
200 1st electrode set
210 2nd electrode set
Si1 to Si3 3rd connection state
Sq1 1st connection state
Sq2 2nd connection state

## Claims

1. A high-frequency treatment apparatus comprising:
a plurality of high-frequency output units outputting high-frequency power;
a plurality of electrodes connected to the high-frequency output units and arranged in a treatment object;
a switching unit provided between the high-frequency output units and the electrodes, and configured to switch which electrode set of at least two of the electrode sets the high-frequency current flows to;
and a control unit controlling the switching unit so as to switch between a first connection state in which the electrode sets different for each of the high-frequency output units are connected and a second connection state in which the electrode sets obtained by combining a plurality of the electrode sets in the first connection state are connected to one of the high-frequency output units in a specific cycle.

2. The high-frequency treatment apparatus according to claim 1, wherein
the second connection state is a connection state in which the electrodes included in the same electrode sets in the first connection state have the same polarity.

3. The high-frequency treatment apparatus according to claim 1, wherein
the second connection state is a connection state in which the electrode set including all of the electrodes is connected to one of the high-frequency output units.

4. The high-frequency treatment apparatus according to any one of claims 1 to 3, further comprising:
an impedance measurement unit connected to the electrodes via the switching unit and configured to measure impedance by outputting an impedance measurement signal,
wherein the control unit controls the switching unit so as to provide a third connection state for connecting the electrode set to the impedance measurement unit between the first connection state and the second connection state.

5. A high-frequency treatment method for performing treatment comprising:
arranging a plurality of electrodes on an object to be treated,
applying a high-frequency current from a plurality of high-frequency output units to one of the electrode sets in which at least two of the electrodes are combined,
wherein the high-frequency treatment method switches at a specific cycle during treatment between a first connection state in which the electrode sets different for each of the high frequency output sections are connected and a second connection state in which the electrode sets obtained by combining a plurality of the electrode sets in the first connection state are connected to one of the high frequency output sections.
